Europäisches Patentamt

European Patent Office

Office européen des brevets

(19)

(11) Numéro de publication: **0 051 526**
**A1**

(12)

# DEMANDE DE BREVET EUROPEEN

(21) Numéro de dépôt: 81401690.3

(22) Date de dépôt: 23.10.81

(51) Int. Cl.³: **A 61 K 9/00**
**A 61 K 31/02, A 61 K 31/025**
**A 61 K 31/08, A 61 K 31/13**
**C 12 N 1/00**

(30) Priorité: 24.10.80 FR 8119878

(43) Date de publication de la demande:
12.05.82 Bulletin 82/19

(84) Etats contractants désignés:
BE IT

(71) Demandeur: Etablissement Public dit : CENTRE
NATIONAL DE LA RECHERCHE SCIENTIFIQUE (CNRS)
15, Quai Anatole France
F-75700 Paris(FR)

(72) Inventeur: Mathis, Gérard
48ter, avenue Anatole France
F-54000 Nancy(FR)

(72) Inventeur: Delpuech, Jean-Jacques
16, rue du Plateau
F-54520 Laxou(FR)

(74) Mandataire: Phélip, Bruno et al,
c/o Cabinet Harlé & Phélip 21, rue de La Rochefoucauld
F-75009 Paris(FR)

(54) Microémulsions aqueuses de fluorocarbures indéfiniment stables à une température donnée, procédé d'obtention et applications.

(57) La présente invention concerne une microémulsion aqueuse de fluorocarbures, indéfiniment stable à une température donnée.

Cette microémulsion est constituée de:

1) un milieu aqueux,

2) un fluorocarbure;

3) un seul agent tensio-actif fluoré non ionique, ledit agent tensio-actif ayant un point de trouble inférieur d'au moins 5 à 10°C à la température de stabilité de la microémulsion lorsque le milieu aqueux est constitué par de l'eau pure.

Application: transporteurs d'oxygène.

EP 0 051 526 A1

Croydon Printing Company Ltd

Microémulsions aqueuses de fluorocarbures indéfiniment
stables à une température donnée, procédé d'obtention
et applications.

La présente invention concerne des microémulsions aqueuses de fluorocarbures. Elle a également pour objet un procédé pour l'obtention de ces
microémulsions et leur application à titre de
transporteurs de gaz, notamment à titre de transporteur d'oxygène.

Il est connu que les fluorocarbures conviennent
comme transporteurs d'oxygène dans les corps vivants
/_L. Clark et al. Triangle XIII n° 2 p. 85 1973_7 et que
des émulsions de fluorocarbures ont déjà été utilisées
comme substituts du sang /Proceedings of the IV[th] International Symposium on Perfluorochemical's blood
substitutes. Ed. Excerpta Medica 1979 _7.

Dans cette application des fluorocarbures,
il est nécessaire de fabriquer des émulsions aqueuses
stables de ces fluorocarbures de manière à véhiculer
l'oxygène dissous dans le fluorocarbure et les sels
et métabolites qui sont normalement présents dans le
plasma. Il est connu que la toxicité des émulsions
varie avec le pourcentage de particules égales ou
supérieures à 0,4 $\mu$m. Par ailleurs, plus lès particules sont fines, plus les émulsions sont efficaces.
Il est important d'avoir des émulsions ayant des
dimensions de particules relativement faibles, en tout
état de cause inférieures à 0,2 $\mu$m, ou mieux encore
inférieures à 0,1 $\mu$m, de manière à ce qu'elles puissent être compatibles avec la circulation dans l'organisme et être administrées par injection ou perfusion
intra-veineuse.

On a déjà proposé des procédés pour l'obtention de telles émulsions. Par exemple,le brevet FR
71.31.983 (publié sous le n° 2.105.287) concerne un procédé pour l'obtention d'une émulsion d'un fluorocarbure pour injection capable de transporter de
l'oxygène, qui consiste à émulsionner une solution
aqueuse d'un fluorocarbure avec un agent tensio-actif,
à centrifuger l'émulsion aqueuse résultante de façon
à diviser les particules de fluorocarbure dans l'émulsion et à stériliser l'émulsion résultante. L'agent
tensio-actif mis en oeuvre est un copolymère polyéthylène-polyoxypropylène ayant un poids moléculaire
compris entre 8200 et 10500, associé à la lécithine
du jaune d'oeuf ou à la lécithine des graines de soja.
Ce procédé nécessite une opération de centrifugation
et ne convient pas à tous les fluorocarbures.

Le brevet FR 74.03.244 (publié sous le numéro
2.246.262) concerne une émulsion stable de composé
fluorocarboné pouvant transporter de l'oxygène. Cette
émulsion stable dans un milieu aqueux physiologiquement acceptable comprend : un composé perfluorocarboné, un phospholipide et au moins un composé d'acide
gras comme adjuvant d'émulsification. Cette émulsion
est préparée par mélange sous pression élevée.

La demande de brevet DE-OS 2.224.182 concerne
un procédé pour la fabrication d'émulsion stable de
particules de fluorocarbures ; ce procédé consiste à
émulsionner sous pression élevée une solution dispersée
contenant un fluorocarbure, un ou plusieurs agents
tensio-actifs non ioniques et un ou plusieurs polyalcools pour former une émulsion de fluorocarbure ayant
une dimension de particules inférieure à 0,2 $\mu$m.

D'autre part, le brevet US 3.778.381 concerne
des microémulsions de fluorocarbures qui nécessitent
pour leur fabrication la mise en oeuvre d'un fluorohalogénocarbure à faible point d'ébullition. Elles
sont obtenues par mélange d'un fluorocarbure avec un
fluorohalogénocarbure, addition du mélange résultant à un système aqueux contenant un agent tensio-actif et agitation jusqu'à ce que la microémulsion soit formée, puis élimination dudit fluorohalogénocarbure par évaporation.

Le brevet FR 73.39.533 (publié sous le numéro 2.249.657) concerne un procédé de préparation de liquides à application biologique et transporteurs d'oxygène. Ce procédé consiste à émulsionner un composé fluoré en présence de deux émulsionnants dont l'un est à caractère hydrophile et l'autre à caractère lipophile. Les émulsionnants mis en oeuvre dans ce procédé sont des agents tensio-actifs fluorés non ioniques du type polyoxyéthylène-fluoro-alkyl-éther.

Les brevets US 4.105.798 et FR 71.537.587 (publié sous le n° 2.313.024) concernent des émulsions aqueuses de composés polycycliques perfluorés, utilisables comme produits de remplacement du sang. Ces composés polycycliques comportent 9 à 18 atomes de carbone et au moins deux carbones tête de pont. Il ne s'agit pas de microémulsions.

La demande de brevet JP 73.36.514, publiée sous le n° 123.184/74 concerne l'obtention d'émulsions aqueuses contenant un dérivé fluorocarboné, comme constituant principal, un agent tensio-actif non ionique et de l'eau. La préparation de ces émulsions nécessite la mise en oeuvre d'une part d'agents stabilisants d'émulsions, d'agents régulateurs de pH ou d'autres agents et d'autre part des moyens mécaniques, tels qu'agitateurs, homogénéisateurs et similaires. Il faut noter que les émulsions préparées selon le procédé décrit dans cette demande de brevet JP 73.36.514 sont stables au maximum pendant un mois, et à 25°C comme le montrent les exemples illustratifs figurant dans cette demande de brevet JP. Par ailleurs on notera que les agents tensio-actifs mis en oeuvre dans ce procédé sont obtenus, conformément aux procédés décrits dans cette demande de brevet JP sous la forme de mélanges ayant une répartition conforme à une courbe de Gauss et non pas sous la forme de produits purs.

Cet état de la technique montre que la fabrication de microémulsions de fluorocarbures est complexe et que les solutions proposées jusqu'à présent sont insatisfaisantes, soit qu'elles fassent appel à des sonications qui libèrent des ions fluor toxiques, soit qu'elles utilisent une homogénéisation mécanique qui nécessite de très grandes précautions pour obtenir des particules suffisamment fines, sans éliminer totalement les risques de libération d'ions fluor.

Il est, de plus, nécessaire d'utiliser des mélanges de divers tensio-actifs dont la composition n'est pas toujours connue avec précision ou de faire appel à un artifice de fabrication (par exemple selon le brevet US 3.778.381). Par ailleurs, ces émulsions sont instables et doivent être conservées congelées jusqu'au moment de l'utilisation.

Dans la thèse présentée à l'Université de NANCY I le 15 décembre 1978 par Monsieur MATHIS, il est indiqué que pour formuler des microémulsions aqueuses avec le minimum de tensio-actif non ionique, il faut choisir un agent tensio-actif fluoré non ionique possédant un point de trouble voisin de 37°C pour réaliser des microémulsions de fluorocarbures utilisables comme substituts du sang. Contrairement aux enseignements de cette thèse qui laissait supposer que le comportement des systèmes ternaires eau/tensio-actifs fluorés non ioniques/fluorocarbures devait être identique à celui des systèmes ternaires contenant des composés hydrogénés homologues, on a trouvé que pour obtenir une microémulsion dans l'eau pure stable à une température donnée ne contenant qu'un seul agent tensio-actif fluoré non ionique, il est indispensable d'utiliser un agent tensio-actif fluoré
non ionique ayant un point de trouble nettement
inférieur à la température de stabilité de la microémulsion souhaitée et plus particulièrement inférieur
d'au moins 5 à 10°C à cette température de stabilité.

On a maintenant trouvé de nouvelles microémulsions qui peuvent être obtenues facilement par
simple mélange de trois constituants définis ci-après
et chauffage jusqu'à une température qui correspond
à la température de stabilité de la microémulsion
désirée.

La présente invention a donc pour objet des
microémulsions aqueuses de fluorocarbures indéfiniment stables à une température donnée qui sont
constituées de :

1) un milieu aqueux ;

2) un fluorocarbure ;

3) un seul agent tensio-actif fluoré non
ionique, ledit agent tensio-actif ayant un point de
trouble inférieur d'au moins 5 à 10°C à la température
de stabilité de la microémulsion lorsque le milieu
aqueux est constitué par de l'eau pure.

Par "microémulsions" on désigne dans la suite
de la description de la présente demande des solutions
isotropes limpides et stables composées d'une solution
aqueuse d'agent tensioactif dans laquelle une huile est
dispersée sous la forme de globules dont la taille
peut varier de quelques dizaines à quelques centaines
d'Angström.

Le terme "milieu aqueux" désigne dans la
présente description aussi bien l'eau pure que les
solutés de remplissage et tous les milieux aqueux
physiologiquement acceptables par exemple les solutions
aqueuses salines ou les solutions aqueuses amylacées.

A titre de tels milieux, on peut citer notamment la solution physiologique de Ringer lactate et les solutions contenant de l'amidon soluble (H.E.S.) ou de la gélatine modifiée ainsi que le "Plasmion".

Dans les microémulsions selon l'invention, on peut utiliser un fluorocarbure quelconque c'est-à-dire un produit organique entièrement ou presque entièrement fluoré, non miscible à l'eau.

Toutefois, dans le cadre d'applications biologiques des microémulsions selon l'invention, c'est-à-dire à titre de transporteurs d'oxygène, il convient d'utiliser des composés fluorés, chimiquement inertés, non toxiques. D'autre part, pour ces applications biologiques, ces composés fluorés ne doivent pas ou pratiquement pas s'accumuler dans l'organisme; ils doivent posséder une tension de vapeur suffisamment faible pour qu'ils n'entrent pas en ébullition dans l'organisme et avoir une aptitude importante à transporter l'oxygène, par exemple d'au moins 30%.

A titre d'exemples de tels fluorocarbures, on pourra citer les fluorocarbures cités dans les brevets FR 71.31.983, 74.06.360 et 74.03.244.

Parmi les fluorocarbures appropriés on citera plus particulièrement :

- les perfluorocycloalcanes ou les perfluoroalkylcycloalcanes ;

- les perfluoroalcanes et les fluoroalcanes partiellement hydrogénés saturés ou insaturés, tels que les produits de formules :

$C_8F_{17}CH=CH_2$; $C_6F_{13}CH=CH_2$ ou $C_8F_{17}C_2H_5$ ;
les produits de formule générale $C_nF_{2n+1}-CH=CHC_mF_{2m+1}$
$\underline{n}$ et $\underline{m}$ étant des nombres entiers,
les composés hétérocycliques perfluorés alkyl-substitués, les amines tertiaires perfluorées, les éthers perfluorés.

A titre d'exemplespréférés de tels fluorocarbures, on mentionnera les composés ci-après :

- perfluorodécaline,
- perfluorotributylamine,
- perfluorotripropylamine,
- perfluorométhyldécaline,
- perfluoroadamantane,
- perfluorodiméthyladamantane et
- perfluorodécane.

A titre d'agents tensio-actifs fluorés non ioniques appropriés aux fins de l'invention, on citera les agents tensio-actifs du type polyoxyéthylène-fluoro-alkyléther.

Lorsque les microémulsions selon l'invention sont utilisées comme transporteurs d'oxygène dans l'organisme il importe de choisir des agents tensio-actifs fluorés non ioniques non toxiques et pharmaceutiquement compatibles et une température de stabilité voisine des températures atteintes par le corps humain, c'est-à-dire comprise entre environ 35 et 40°C.

Une classe particulièrement préférée d'agents tensio-actifs du type polyoxyéthylène-fluoro-alkyléther est celle des composés répondant à la formule générale :

$$R_F(CH_2) (OC_2H_4)_n OH,$$

dans laquelle $R_F$ est une chaîne fluorocarbonée ou fluorocarbonée partiellement hydrogénée,

$\underline{n}$ est un nombre entier au moins égal à 1.

Ces composés peuvent être obtenus par le procédé qui fait l'objet de la demande de brevet FR 80.22.874 déposée le 24 Octobre 1980 au nom du demandeur et intitulée "procédé pour la synthèse de composés hydrogénés et/ou fluorés du type polyoxyéthylène-alkyl-éther, composés de ce type et application à titre d'agents tensio-actifs non ioniques". Parmi ces composés, on citera tout particulièrement les composés de formules :

$C_7F_{15}CH_2(OC_2H_4)_4OH$ ; $C_7F_{15}CH_2 (OC_2H_4)_5 OH$ ; $C_7F_{15}CH_2$
$(OC_2H_4)_6 OH$ ; $C_6F_{13}CH_2(OC_2H_4)_3OH$ ; $C_6F_{13}CH_2 (OC_2H_4)_4 OH$;
$C_6F_{13}CH_2(OC_2H_4)_5OH$ et $C_6F_{13}CH_2 (OC_2H_4)_6OH$

On a trouvé de façon surprenante que l'on pouvait obtenir une microémulsion stable à une température donnée en mélangeant simplement des quantités adéquates des trois constituants définis ci-dessus et en chauffant le mélange résultant jusqu'à la température de stabilité de la microémulsion désirée.

On a en effet trouvé qu'il existe des zones de concentrations relatives de ces trois constituants pour lesquelles on obtient, à une température donnée, une microémulsion stable. Si la température de la microémulsion obtenue est modifiée, par exemple au cours du transport ou lors de la conservation au réfrigérateur ou de la stérilisation, la microémulsion limpide se reforme de la même façon par agitation à la température de stabilité de la microémulsion considérée. Cette microémulsion est stable indéfiniment dans le temps au voisinage de sa température de stabilité. On a également trouvé que pour un fluorocarbure donné la zone de stabilité de la micro-émulsion à une température donnée se déplace vers les fortes teneurs en fluorocarbure et les faibles teneurs en agent tensio-actif lorsque l'on diminue l'hydrophilie de l'agent tensio-actif. Il se produit un effet identique lorsque l'on ajoute des sels ou des macromolécules de type amidon soluble (H.E.S.), ou gélatine modifiée.

Il convient d'utiliser aux fins de l'invention, lorsque le milieu aqueux est de l'eau pure, un agent tensio-actif fluoré non ionique présentant un point de trouble inférieur à la température de stabilité de la micro-émulsion désirée et plus particulièrement inférieur d'au moins 5 à 10°C à cette température de stabilité.

L'écart entre le point de trouble de l'agent tensio-actif et de la température de stabilité dépend d'une part du fluorocarbure, par exemple, dans le cas de fluorocarbures linéaires de même type, de la longueur de la chaîne fluorocarbonée et d'autre part, des différents additifs présents dans la phase aqueuse (sels, macromolécules).

Comme on l'a indiqué ci-dessus, cet écart doit être d'au moins 5 à 10°C lorsque le milieu aqueux est constitué uniquement d'eau pure. Par contre, lorsqu'on ajoute des sels ou des macromolécules dans le milieu aqueux constitué d'eau pure, le tensio-actif, nécessaire pour obtenir une microémulsion occupant une position sensiblement comparable à celle de la micro-émulsion sans sel ni macromolécule (c'est-à-dire ayant sensiblement la même concentration en fluorocarbure et la même température de stabilité), doit être plus hydrophile (HLB plus élevé) c'est-à-dire avoir un point de trouble supérieur à celui du tensio-actif mis en oeuvre pour obtenir la micro-émulsion dans de l'eau pure. Si on utilise le même agent tensio-actif dans les deux cas, la température de stabilité de la microémulsion contenant la même proportion de fluorocarbure et d'agent tensio-actif mais contenant plus de sels ou de macromolécules, sera, si la concentration en additifs est notable, inférieure à celle de la microémulsion formée avec de l'eau pure.

Il sera aisé à l'homme de l'art de déterminer, pour un fluorocarbure et un agent tensio-actif fluoré non ionique donnés, par des essais de routine, la température à laquelle se forme la microémulsion.
Pour obtenir une microémulsion dudit fluorocarbure
stable à une autre température, il y aura lieu de
modifier les proportions relatives des trois constituants du mélange ou de choisir un agent tensioactif
fluoré non ionique ayant une hydrophilie supérieure
ou inférieure à celui utilisé pour la détermination
ci-dessus, suivant que la température de stabilité de
la microémulsion souhaitée est supérieure ou inférieure à celle définie ci-dessus.

A titre indicatif on précisera que lorsque le tensio-actif fluoré non ionique est du type polyoxyéthylène-fluoro-alkyl-éther, ayant un nombre bien défini
de motifs éther, on peut obtenir des microémulsions de
perfluorodécaline, du fluorocarbure de formule
$C_8F_{17}CH=CH_2$ ou de perfluorotributylamine stables à
37°C et pouvant incorporer des quantités notables
d'oxygène, en utilisant des agents tensio-actifs de ce
type ayant un point de trouble inférieur ou voisin de
0°C. Des exemples de tels tensio-actifs sont ceux ayant
6 à 7 atomes de carbone dans le radical $R_F$ et 5 ou 6
motifs éther.

Il importe alors de déterminer pour chaque fluorocarbure et pour un agent tensio-actif donné la zone de
concentrations qui procure une microémulsion stable
à une température donnée.

Il sera aisé à l'homme de l'art de déterminer ces
zones par des essais de routine et d'établir pour
chaque fluorocarbure le diagramme ternaire comportant
ladite zone de concentration appropriée pour un agent
tensio-actif donné.

Pour déterminer ces zones de concentration, on forme par exemple des mélanges binaires avec deux des trois constituants de la microémulsion et, à la température souhaitée de stabilité, on ajoute des quantités croissantes du troisième constituant et on note la quantité qui permet l'obtention de la microémulsion limpide ; cette quantité constitue la limite inférieure de la zone de concentration pour le système binaire considéré. On augmente ensuite la concentration du troisième constituant jusqu'à la démixtion pour fixer la limite supérieure de ladite zone. Ensuite, on fait varier la concentration relative du système binaire de départ et on opère de la même façon. Ce mode opératoire est répété un nombre suffisant de fois pour obtenir avec précision la zone de concentration appropriée et on établit ainsi le diagramme pour chaque système ternaire correspondant.

Les figures 1 à 4 annexées sont les diagrammes spécifiques pour les systèmes ci-après :

Fig. 1 : eau/perfluorodécaline/$C_7F_{15}CH_2(OC_2H_4)_5OH$

Fig. 2 : eau/$C_8F_{17}CH = CH_2$ / $C_7F_{15}CH_2(OC_2H_4)_6OH$

Fig. 3 : solution de Ringer lactée /$C_8F_{17}CH = CH_2$/ $C_7F_{15}CH_2(OC_2H_4)_6OH$

Fig. 4 : eau/perfluorodécaline / $C_6F_{13}CH_2(OC_2H_4)_5OH$

Ces diagrammes ont été déterminés selon le mode opératoire ci-dessus ; les zones hachurées constituent les zones de concentrations appropriées pour des températures de stabilité d'environ 37°C et 25°C respectivement.

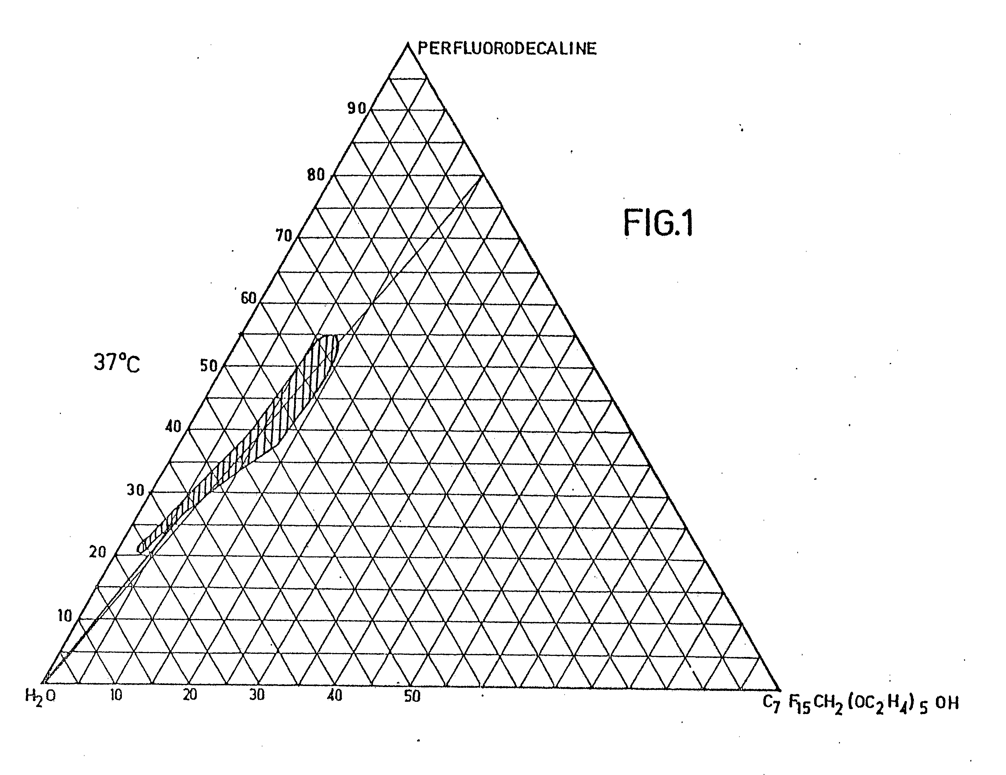

Ainsi, comme on peut le voir sur la figure 1, pour obtenir une microémulsion stable à environ 37°C avec la perfluorodécaline et l'agent tensio-actif de formule $C_7F_{15}CH_2(OC_2H_4)_5OH$ il faut utiliser en poids : environ 0 à 55 % de la perfluorodécaline, environ 0 à 15 % de l'agent tensio-actif $C_7F_{15}CH_2(OC_2H_4)_5OH$, le complément étant de l'eau.

12

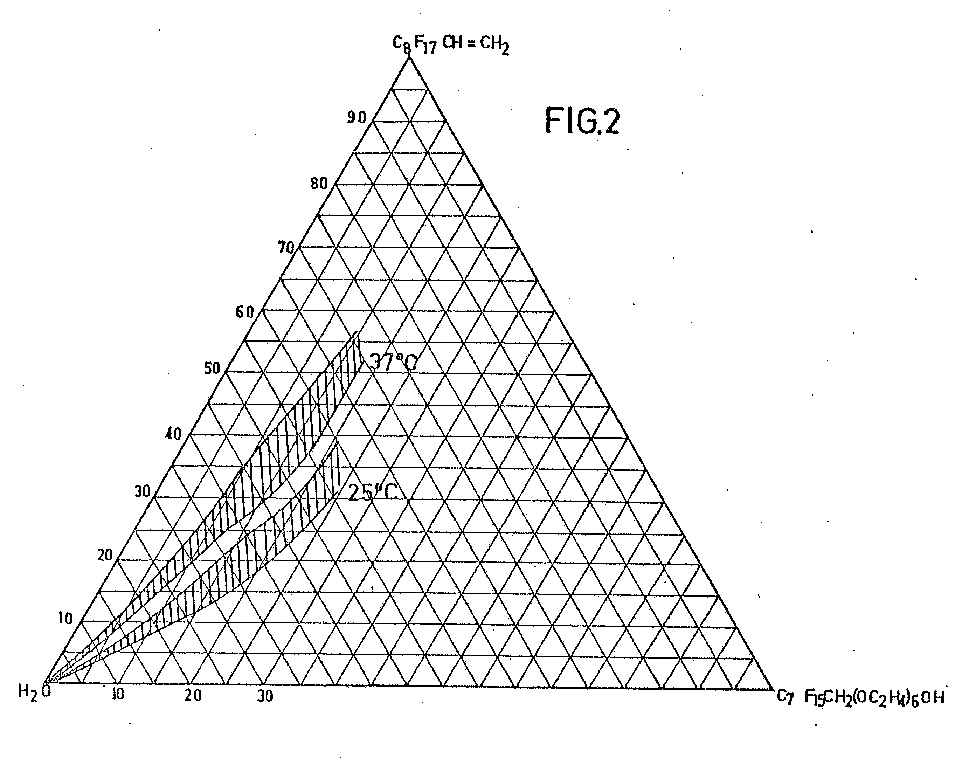

Pour obtenir une microémulsion stable à environ 37°C avec le fluorocarbure de formule $C_8F_{17}CH=CH_2$ (voir figure 2) il faudra utiliser 0 à 60% dudit fluorocarbure, 0 à 20% de l'agent tensio actif $C_7F_{15}CH_2(OC_2H_4)_6OH$, le complément étant de l'eau.

Pour obtenir une microémulsion stable à environ 25°C avec les mêmes constituants, il faudra utiliser 0 à 45% dudit fluorocarbure, 0 à 25% de l'agent tensio-actif $C_7F_{15}CH_2(OC_2H_4)_6OH$, le complément étant de l'eau.

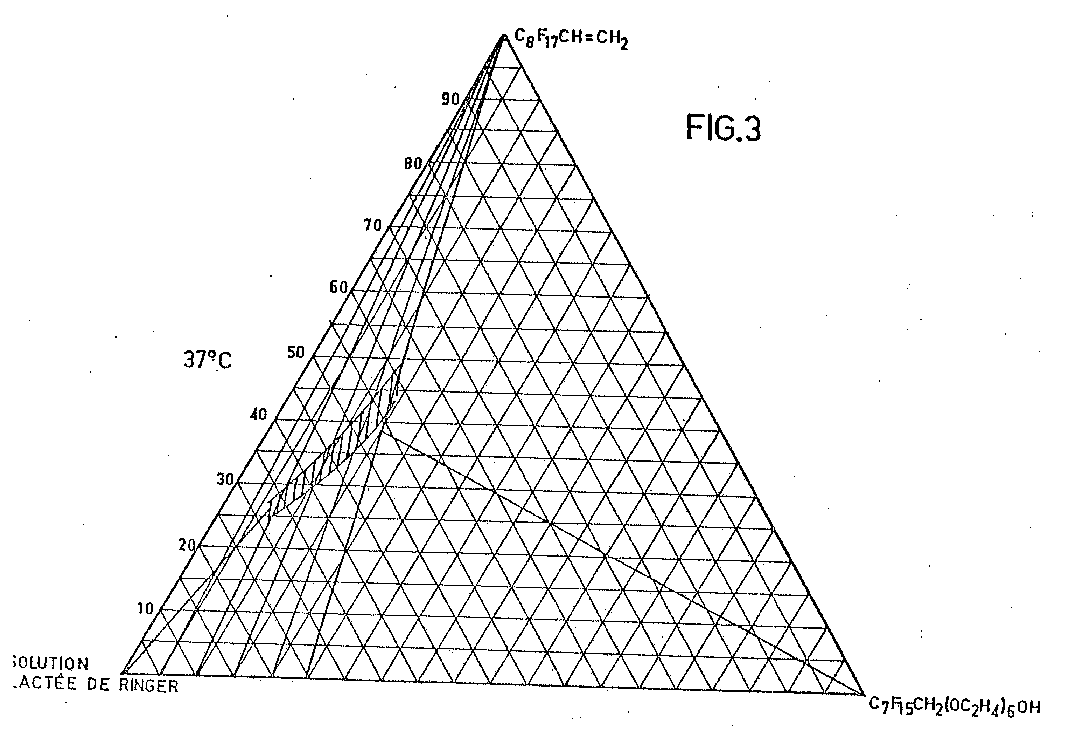

Pour obtenir une microémulsion stable à environ 47°C avec le fluorocarbure de formule $C_8F_{17}CH=CH_2$, le milieu aqueux étant constitué de la solution lactée de Ringer il faudra utiliser (voir figure 3):

0 à 55% de $C_8F_{17}CH=CH_2$

0 à 20% de l'agent tensioactif de formule $C_7F_{15}CH_2(OC_2H_4)_6OH$, le complément étant constitué de la solution de Ringer-lactate.

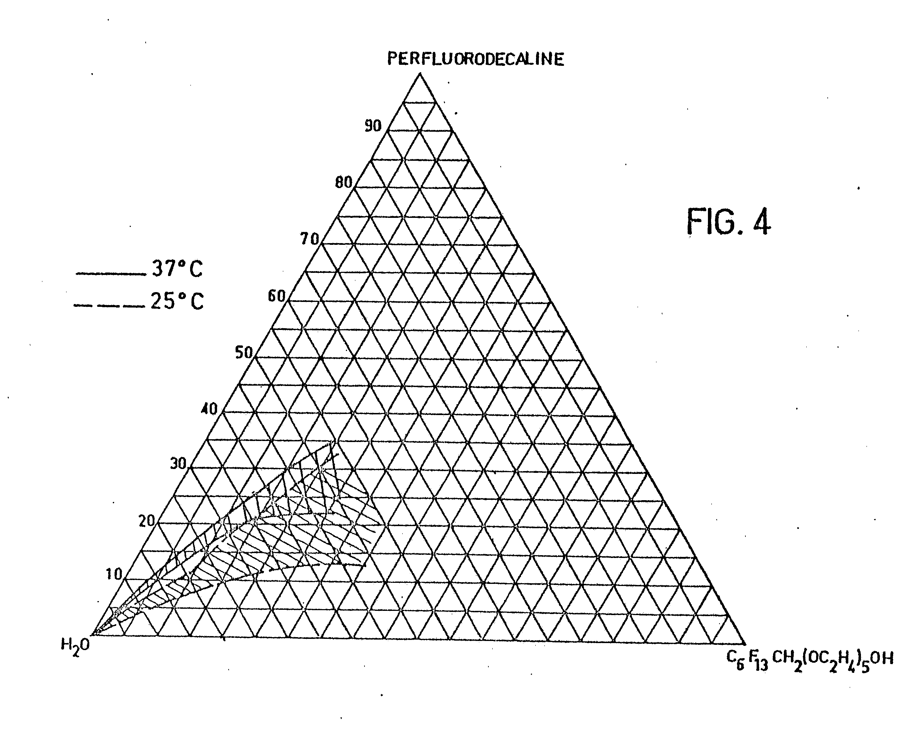

Pour obtenir une microémulsion stable à 37°C de perfluorodécaline dans l'eau (voir figure 4) il convient d'utiliser :

0 à 45% de perfluorodécaline

0 à 50% de l'agent tensio-actif $C_6F_{13}CH_2(OC_2H_4)_5OH$ le complément étant de l'eau.

Pour obtenir une microémulsion stable à 25°C, il faudra utiliser pratiquement les mêmes gammes comme on peut le voir sur la figure 4.

Lorsque l'on a déterminé le diagramme ternaire d'un système eau/fluorocarbure/agent tensio-actif, à une température donnée, par exemple à 37°C, il est alors possible de prévoir l'évolution de la position de la zone de la microémulsion, à cette même température, lorsque l'on modifie l'hydrophilie de l'agent tensio-actif mis en oeuvre. On a trouvé en effet que lorsque l'on utilise un agent tensio- actif second plus hydrophile que l'agent actif premier, utilisé pour établir la zone de microémulsion d'un fluorocarbure donné à une température donnée, on obtient à la même température, une zone de microémulsion déplacée vers la base $H_2O$ / agent tensio-actif du diagramme c'est-à-dire que le rapport massique fluorocarbure / agent tensio-actif second est plus faible que le rapport massique fluorocarbure/agent tensio-actif premier. Inversement, lorsqu'on utilise un agent tensio-actif second moins hydrophile que l'agent tensio-actif premier la zone de microémulsion s'éloigne de la base $H_2O$/agent tensio-actif.

A cet effet on peut se référer aux diagrammes des figures 1 et 4 ; pour le système ternaire $H_2O$/ perfluorodécaline / $C_7F_{15}CH_2(OC_2H_4)_5OH$, la zone de microémulsion à 37°C existe dans une gamme de composition au voisinage d'un rapport fluorocarbure/ agent tensio-actif voisin de 80/20, soit 4 alors que pour le système ternaire $H_2O$/perfluorodécaline/ $C_6F_{13}$ $CH_2(OC_2H_4)_5OH$ la zone de microémulsion à 37°C est au voisinage d'un rapport fluorocarbure / agent tensio-actif compris entre 65/35 et 60/40, soit de 1,85 à 1,50.

On notera que l'augmentation de l'hydrophilie d'un agent tensio-actif donné peut être obtenue soit par augmentation du nombre de motifs éther, soit par diminution du nombre de motifs $CF_2$.

Comme on l'a indiqué précédemment la présence de sels ou de macromolécules modifie la position de la zone de microémulsion pour un système ternaire donné. On a trouvé que la présence de sels ou de macromolécules de type gélatine modifiée dans la phase aqueuse, a un effet comparable à celui engendré par une diminution de l'hydrophilie du tensio-actif, ce qui s'explique par un effet de "salting out" ou de destructuration de l'eau bien connu pour les tensio-actifs non ioniques hydrogénés (voir à cet effet les références ci-après :

- Friberg S. and Lapczynska I. Progr. Colloïd Polymer
Sci. 56, 16-20, 1975.
- Shinoda K., Takeda H., J. Coll. Interf. Sci. 32,
p. 642, 1970).

Ainsi, si l'on examine les microémulsions de perfluorodécaline obtenues à partir d'une solution de 20 % de $C_6F_{13}CH_2(OC_2H_4)_5OH$ dans l'eau ou le Ringer Lactate, on remarque que dans le premier cas, la microémulsion à 37°C est obtenue pour des compositions de perfluorodécaline comprises entre 21 et 27 % alors que dans le deuxième cas elle se situe entre 24 et 29 % de perfluorodécaline, cet effet étant comparable à une diminution de l'hydrophilie du tensio-actif.

De la même façon, avec les microémulsions de perfluorodécaline obtenues à partir de solutions de 20 % de $C_7F_{15}CH_2(OC_2H_4)_6OH$ dans l'eau ou le "Plasmion", on remarque que dans le premier cas la microémulsion à 37°C est obtenue pour des compositions en perfluorodécaline comprises entre 9 et 16% alors que dans le deuxième cas, la microémulsion est obtenue pour des compositions en perfluorodécaline comprises entre 14 et 20 % , ce comportement étant encore une fois parallèle à celui qui serait engendré par une diminution de l'hydrophilie du tensio-actif. Ces résultats d'essais sont indiqués respectivement sur les diagrammes ternaires :

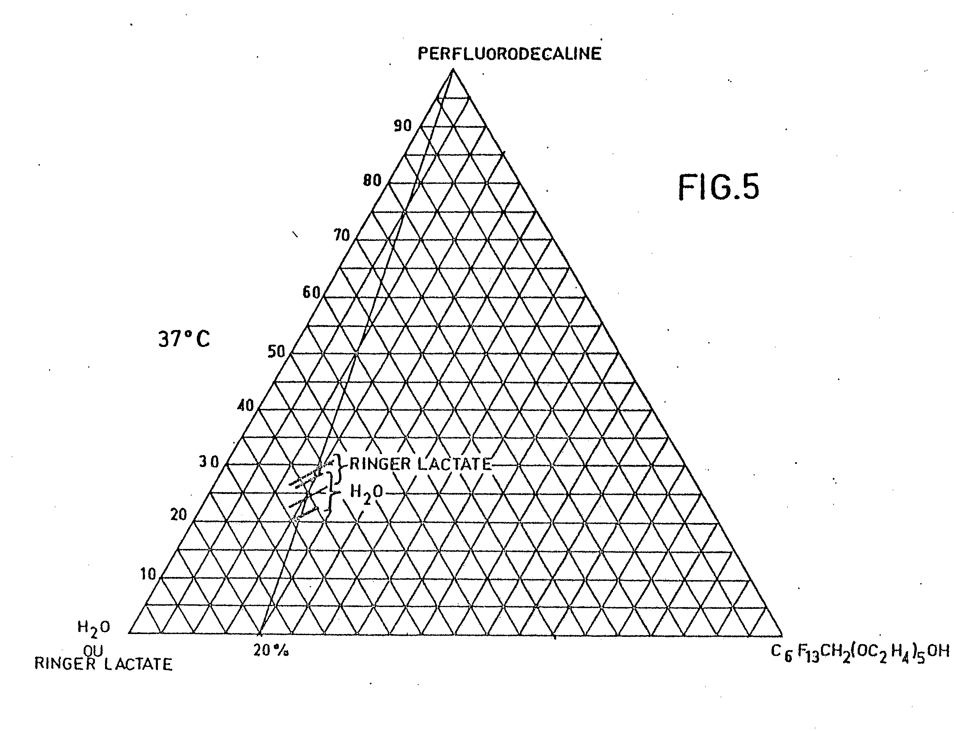

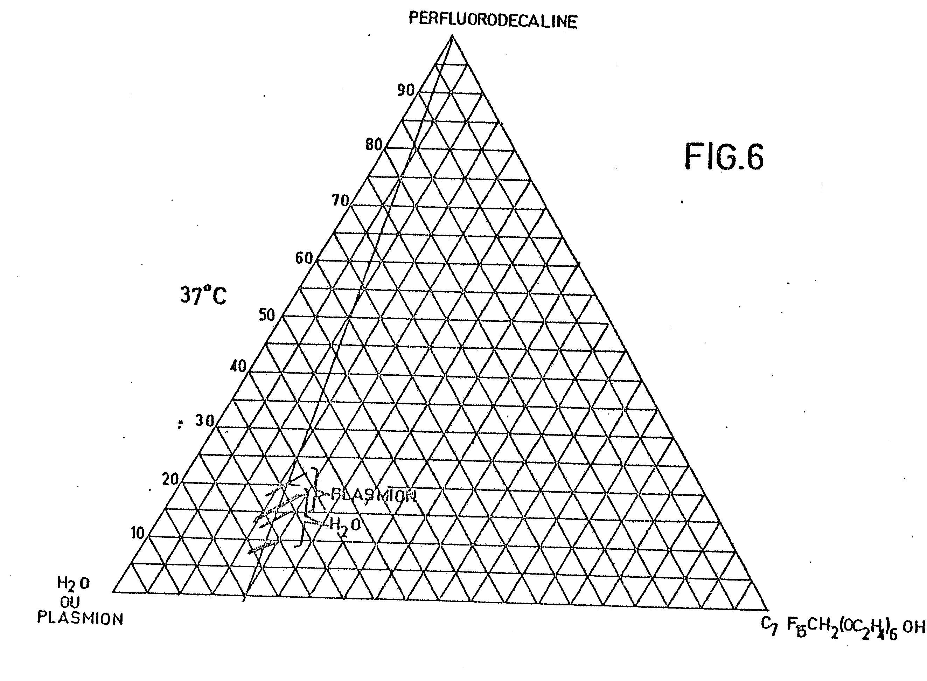

- eau ou Ringer-lactate/perfluorodécaline/$C_6F_{13}CH_2$ $(OC_2H_4)_5OH$ de la figure 5 annexée et
- eau ou "Plasmion"/perfluorodécaline/$C_7F_{15}CH_2(OC_2H_4)_6$ OH de la figure 6 annexée .

On notera que le produit connu sous la dénomination commerciale "Plasmion" est un substitut du plasma sanguin constitué par l'addition d'une macromolécule (gélatine fluide modifiée) à un soluté ionique équilibré du type Ringer-lactate.

L'utilisation des émulsions de fluorocarbures selon l'art antérieur comme substituts du sang,nécessite le mélange de l'émulsion aqueuse de fluorocarbure avec des solutés de remplissage. Selon l'invention on peut obtenir des microémulsions prêtes à l'emploi,c'est-à-dire des microémulsions contenant le fluorocarbure comme transporteurs d'oxygène et le soluté de remplissage qui constitue le milieu aqueux utilisé pour la fabrication de la microémulsion. Il peut être avantageux de fabriquer des formulations pharmaceutiques constituant des unités de micro-émulsions de 300 ml par exemple,puisqu'il est courant d'utiliser dans les transfusions sanguines des unités de sang ou de substituts de sang de 300 ml. Ces formulations pharmaceutiques sont réalisées selon des moyens connus de l'homme de l'art pour satisfaire à la législation pharmaceutique. Des exemples d'unités de 300 ml seront donnés ci-après dans les exemples illustratifs.

La taille et la forme des particules des microémulsions selon l'invention peuvent être déterminées par diffusion des neutrons aux petits angles(Small angle neutron scattering). L'examen de la courbe de diffusion de la microémulsion permet de déterminer la valeur du nombre d'agrégation et du rayon de giration des particules à partir des expériences effectuées aux petits angles sur les microémulsions très diluées (région riche en eau).

La comparaison de la courbe obtenue aux plus grands angles avec un modèle théorique permet de déterminer la forme des particules.

Pour le système ternaire $H_2O/ C_8F_{17}CH=CH_2/C_7F_{15}CH_2(OC_2H_4)_6OH$ représenté sur le diagramme de la figure 2 on a trouvé,qu'à la température de 25°C pour un rapport massique fluorocarbure/ agent tensioactif de 1,4 les particules étaient des éllipsoïdes possédant un nombre d'agrégation voisin de 2000 et un grand axe de 200 Å.

On a par ailleurs constaté que la taille et la forme des particules ne varient pas lors de la dilution de la microémulsion dans un milieu aqueux. D'autre part, on a constaté que la stabilité de la microémulsion n'est pas altérée lors de la dilution dans un milieu aqueux. Ainsi, lors de l'utilisation des microémulsions à titre de substituts du sang, leur stabilité et la taille des particules de celles-ci ne seront pas altérées dans l'organisme, puisqu'au cours de la transfusion de ces microémulsions il se produit une dilution de celles-ci dans le sang du patient. Des essais effectués avec des microémulsions selon le diagramme de la figure 2 ont en effet montré que, lors de la dilution en milieu aqueux de celles-ci, la dimension des particules n'était pas modifiée et que la stabilité de ces microémulsions était conservée.

La présente invention permet de pallier les difficultés de mise en émulsion et de mauvaise stabilité des solutions aqueuses de fluorocarbures utilisées jusqu'à présent.

Les microémulsions de l'invention trouvent une application particulière dans le domaine médical, notamment à titre de transporteur d'oxygène. Dans ce cas particulier, il importe seulement d'utiliser un agent tensio-actif non toxique chimiquement inerte, ne s'accumulant pas dans l'organisme.

Les microémulsions selon l'invention ont une capacité de fixation de l'oxygène meilleure que celle des émulsions de l'art antérieur, ce qui permet de travailler à des pressions d'oxygène plus faibles comparativement à celles nécessaires avec les émulsions de l'art antérieur, ces pressions d'oxygène plus faibles se rapprochant davantage des conditions physiologiques.

Les microémulsions selon l'invention sont également appropriées pour la perfusion d'organes isolés, le transport d'azote ou de gaz carbonique ou comme additifs dans les milieux de cultures de bactéries aérobies ,ou anaérobies.

L'invention va être maintenant décrite plus en détail dans les exemples illustratifs ci-après. Tous les pourcentages sont en poids aussi bien dans les exemples ci-après que sur les diagrammes des figures 1 à 6.

EXEMPLE 1

Préparation d'une microémulsion

$H_2O/C_8F_{17}CH = CH_2/C_7F_{15}CH_2 (OC_2H_4)_6 OH$ stable à 37°C.

On a déterminé la zone de concentrations spécifique de la stabilité à 37°C du système ternaire ci-dessus en opérant selon le mode opératoire défini précédemment et on a établi le diagramme ternaire de la figure 2.

Dans un tube à essais on a ajouté :

3,5 g de $C_8F_{17}CHCH_2$

5,2 g d'eau

1,3 g de $C_7F_{15}CH_2 (OC_2H_4)_6 OH$ (point de trouble $<$ 0°C)

la solution a été portée à 37°C dans un thermostat ; après agitation manuelle, on a obtenu une solution limpide stable indéfiniment à 37°C.

On a refroidi la microémulsion ainsi obtenue jusqu'à 25°C. Le mélange ternaire était alors sous la forme deux phases, une phase supérieure limpide, une phase inférieure laiteuse. On a ensuite porté ce mélange à 37°C et on a obtenu à nouveau une micro-émulsion stable qui présentait les caractéristiques identiques à celles de la microémulsion obtenue après mélange des trois constituants et chauffage à 37°C.

De la même manière on a mélangé dans un tube

18

à essais :

$4,5$ g de $C_8F_{17}CHCH_2$

4 g d'eau

$1,5$ g de $C_7F_{15}CH_2(OC_2H_4)_6OH$

la solution a été portée à 37°C dans un thermostat ; après une agitation manuelle, on a obtenu une solution limpide stable indéfiniment à 37°C.

On a également obtenu une microémulsion stable à 37°C en mélangeant selon le mode opératoire ci-dessus $2,5$ g de $C_8F_{17}CHCH_2$, $6,5$ g d'eau et 1 g de $C_7F_{15}CH_2(OC_2H_4)_6OH$. On a déterminé que cette microémulsion dissolvait 12 ml d'oxygène pour 100 ml de microémulsion à 37°C.

EXEMPLE 2

Préparation d'une microémulsion

Ringer lactate $/C_8F_{17}CH = CH_2/C_7F_{15}CH_2(OC_2H_4)_6OH$ stable à 37°C.

On a déterminé la zone de concentration spécifique de stabilité à 37°C du système ternaire ci-dessus en opérant selon le mode opératoire défini précédemment et on a établi une partie du diagramme ternaire représenté sur la figure 3.

Dans un tube à essai on a ajouté :

$3,3$ g $C_8F_{17}CHCH_2$

$5,7$ g de solution lactée de Ringer

1 g de $C_7F_{15}CH_2(OC_2H_4)_6OH$

le mélange a été porté à 37°C ; après agitation manuelle on a obtenu une solution limpide stable indéfiniment à 37°C.

EXEMPLE 3

Préparation d'une microémulsion

eau/fluorodécaline/$C_7F_{15}CH_2(OC_2H_4)_5OH$

On a déterminé la zone de concentration spécifique de la stablilité à 37°C du système ternaire ci-dessus en opérant selon le mode opératoire défini précédemment et on a établi le diagramme ternaire de la figure 1.

Dans un tube à essai on a ajouté :

3,8 g de perfluorodécaline

5,3 g d'eau

0,9 g de $C_7F_{15}CH_2(OC_2H_4)_5OH$.

Le mélange a été porté à 37°C ; après agitation manuelle, on a obtenu une solution limpide stable indéfiniment à 37°C.

On a également obtenu une microémulsion indéfiniment stable à 37°C en mélangeant :

4,16 g de perfluorodécaline

2,88 g d'eau

0,96 g de $C_7F_{15}CH_2(OC_2H_4)_5OH$

On a déterminé que cette microémulsion dissolvait 17,5 ml d'oxygène pour 100 ml de microémulsion à 37°C.

EXEMPLE 4

Microémulsion système $C_7F_{16}/H_2O/C_7F_{15}CH_2(OC_2H_4)_6OH$

On a utilisé un mélange constitué de :

1,3 g $C_7F_{15}CH_2(OC_2H_4)_6OH$

1,2 g $C_7F_{16}$

7,5 g $H_2O$

On a opéré selon le mode opératoire défini précédemment et on a obtenu une microémulsion stable à 37°C.

EXEMPLE 5

Microémulsion système perfluorotributylamine/$H_2O/C_7F_{15}CH_2(OC_2H_4)_5OH$

On a utilisé un mélange constitué de :

1,8 g perfluorotributylamine

1,2 g $C_7F_{15}CH_2(OC_2H_4)_5OH$

7 g $H_2O$

on a obtenu une microémulsion stable à 37°C.

EXEMPLE 6

Microémulsion système perfluorodécaline/$H_2O/C_6F_{13}CH_2(OC_2H_4)_5OH$.

On a utilisé un mélange constitué de :

2,5 g de perfluorodécaline

6 g d'eau

1,5 g de $C_6F_{13}CH_2(OC_2H_4)_5OH$

Après avoir porté le mélange à 37°C et agité manuellement on a obtenu une microémulsion indéfiniment stable.

EXEMPLE 7 :

Système perfluorodécaline/Ringer lactate/$C_6F_{13}CH_2$ $(OC_2H_4)_5OH$

On a utilisé un mélange constitué de :

2,9 g de perfluorodécaline

5,6 g de Ringer Lactate

1,5 g de $C_6F_{13}CH_2(OC_2H_4)_5OH$

On a porté le mélange à 37°C et après agitation manuelle, une microémulsion indéfiniment stable a été obtenue.

EXEMPLE 8 :

Système perfluorodécaline/Plasmion/$C_7F_{15}CH_2(OC_2H_4)_6OH$

On a utilisé un mélange constitué de :

1,9 g de perfluorodécaline

6,5 g de Plasmion

1,6 g de $C_7F_{15}CH_2(OC_2H_4)_6OH$

Après agitation manuelle du mélange à 37°C, une micro-émulsion limpide indéfiniment stable à 37°C a été obtenue.

EXEMPLE 9

Système perfluorodécaline/$H_2O$/$C_7F_{15}CH_2(OC_2H_4)_6OH$

On a utilisé un mélange constitué de :

1,5 g de perfluorodécaline

6,7 g $H_2O$

1,8 g $C_7F_{15}CH_2(OC_2H_4)_6OH$.

Après agitation manuelle à 37°C, une microémulsion limpide et indéfiniment stable à 37°C a été obtenue.

EXEMPLE 10

$C_8F_{17}CH_2CH_3$/Ringer Lactate/$C_7F_{15}CH_2(OC_2H_4)_6OH$

On a utilisé un mélange constitué de :

1,1 g $C_8F_{17}CH_2CH_3$

4,17 g Ringer Lactate

0,76 g $C_7F_{15}CH_2(OC_2H_4)_6OH$

Après agitation manuelle à 37°C, une microémulsion indéfiniment stable à 37°C a été obtenue.

EXEMPLE 11

Préparation d'unités de 300 ml

En opérant selon le mode opératoire défini dans l'exemple 1 on a établi les zones de concentrations appropriées pour obtenir des microémulsions indéfiniment stables avec les systèmes ternaires ci-après :

$C_8F_{17}CH = CH_2$ / $C_7F_{15}CH_2(OC_2H_4)_6OH$/Ringer-Lactate

perfluorodécaline/$C_6F_{13}CH_2(OC_2H_4)_5OH$/Ringer-Lactate

perfluorodécaline/$C_7F_{15}CH_2(OC_2H_4)_6OH$/"Plasmion"

et on a formé les unités de 300 ml définies ci-après :

(1) 130,4 g de $C_8F_{17}CH = CH_2$

35,5 g de $C_7F_{15}CH_2(OC_2H_4)_6OH$

La solution a été complétée à 300 ml avec du Ringer Lactate.

(2) 108 g de perfluorodécaline 55,9 g de $C_6F_{13}CH_2(OC_2H_4)_5OH$

La solution a été complétée à 300 ml avec du Ringer Lactate.

(3) 56,5 g $C_7F_{15}CH_2(OC_2H_4)_6OH$

69,9 g perfluorodécaline

La solution a été complétée à 300 ml avec du "Plasmion" Essais d'hémocompatibilité in vitro

On a recherché si les microémulsions selon l'invention avaient une action sur le sang humain en effectuant un thromboélastogramme, en mesurant le temps de thrombine diluée et en évaluant l'hémolyse.

A - protocole d'essais :

- le sang humain était du sang de sujets normaux, prélevé le jour de l'essai, en flacon siliconé, et sur citrate à 9 %, dans le rapport du 1/20.

- les produits (microémulsions ou témoin) mis en présence de ce sang, en proportions variables, ont permis, après 10 minutes d'incubation à la température du laboratoire, de pratiquer un thromboélastogramme sur sang total, afin de mettre en évidence une action éventuelle sur la coagulation globale selon le mode opératoire décrit par RABY C. dans Biologie des Hémorragies et des Thromboses 1 vol., Masson et Cie, Edit. Paris 1966 p.187

- un temps de thrombine diluée a été effectué sur les plasmas des sangs ayant été en contact avec les microémulsions selon l'invention pendant 30 minutes à la température du laboratoire selon le mode opératoire décrit par RABY C. à la page 119 de l'ouvrage cité ci-dessus.

- L'hémolyse a été évaluée sur ces plasmas après centrifugation du sang à 2.000 g pendant 15 minutes.

B - Microémulsions testées et témoin

Dans ces essais on a utilisé les microémulsions selon l'invention définies ci-après :

Microémulsion R 716 C 81

$$\text{tensio actif } C_7F_{15}CH_2(OC_2H_4)_6OH \quad : 12,6\% \text{ en poids /poids}$$

$$\text{fluorocarbure } C_8F_{17}CH_2CH_{13} \quad 18,3 \% \text{ en poids/poids}$$

solution de Ringer Lactate : 69,1% en poids/poids

Microémulsion R 716 C 82

$$\text{tensio actif } C_7F_{15}CH_2(OC_2H_4)_6OH \quad : 9,80 \% \text{ en poids/poids}$$

Fluorocarbure : $C_8F_{17}CH=CH_2$      : 35,90% en poids/poids

Solution de Ringer Lactare      : 54,30% en poids/poids

Comme témoin on a utilisé l'agent tensio-actif seul EF 716 c'est-à-dire le produit de formule $C_7F_{15}CH_2(OC_2H_4)_6OH$, en solution dans de l'éthanol, puisque ce tensio-actif n'est pas soluble dans les solutés physiologiques. A des fins de comparaison, on a également utilisé les microémulsions ci-dessus d'une part en présence d'éthanol et d'autre part en l'absence d'éthanol.

Dans la détermination du temps de thrombine on a utilisé de l'eau physiologique comme témoin.

C- Résultats :

- En présence d'éthanol (V/V), une solution limpide a été obtenue avec l'agent tensio-actif. Celle-ci, ajoutée dans les proportions de 0,2 ml à 1,8 ml d'eau physiologique, a donné, après agitation, une émulsion laiteuse, instable, s'éclaircissant en 15 minutes environ, tant à la température du laboratoire qu'à 37°C.

- Cette émulsion, mise en présence de quatre échantillons de sang humain, à raison de 0,1 ml pour 0,9 ml de sang, a permis d'effectuer les essais définis ci-dessus.

- Aucune action directe n'a été observée après adjonction du tensio-actif $C_7F_{15}CH_2(OC_2H_4)_6OH$ à du sang normal. La fluidité n'a pas été altérée, et il n'existait aucune trace d'hémolyse.

- Après recalcification, déclenchant la coagulation du sang total, et dont les phénomènes sont enregistrés par le thromboélastogramme, on a constaté un léger raccourcissement de la constante "r", un allongement de "k" et une diminution de l'amplitude "amx". Ces modifications mineures n'ont pas de signification particulière.

Microémulsions R 716 C 81 - R 716 C 82

1) En présence d'éthanol

Des essais similaires à ceux pratiqués avec l'agent tensio-actif en présence d'éthanol, ont donné des résultats identiques.

2) Sans éthanol

Ces émulsions, troubles à la température du laboratoire, ont donné des microémulsions limpides lorsqu'elles ont été portées quelques instants à 37°C.

- Utilisés purs et dilués au 1/10 en eau physiologique, sans adjonction d'éthanol, et mis en présence de sang humain dans les mêmes proportions que précédemment et pendant un laps de temps identique, R 716 C 81 et R 716 C 82 ont produit les mêmes effets que ceux déjà observés avec éthanol.

- Le temps de·thrombine diluée, pratiqué dans cet essai, est très légèrement allongé par rapport au témoin constitué dans cet essai par de l'eau physiologique, le R 716 C 82 produisant les temps les plus longs. Ces temps de thrombine diluée sont indiqués dans le tableau I ci-après.

TABLEAU I

Temps de thrombine

|  | Sang I | Sang II |
|---|---|---|
| Témoin eau physiologique | 26" | 28" |
| R 716 C 81 non dilué | 28" | 31" |
| R 716 C 81 dilué 1/10 | 28" | 29" |
| R 716 C 82 non dilué | 32" | 33" |
| R 716 C 82 dilué 1/10 | 31" | 32" |

Ces essais montrent que l'agent tensio-actif seul et les microémulsions  R 716 C 81 et R 716 C 82 sont sans effet _in vitro_ sur le sang humain, non coagulé, ne produisent pas d'activité semblable à la thrombine et ne semblent pas apporter de modifications au niveau de la membrane érythrocytaire, étant donné l'absence d'hémolyse.

REVENDICATIONS

1. Microémulsion aqueuse de fluorocarbure indéfiniment stable à une température donnée, caractérisée en ce qu'elle est constituée de :

1) un milieu aqueux;

2) un fluorocarbure;

3) un seul agent tensio-actif fluoré non ionique, ledit agent tensio-actif ayant un point de trouble inférieur d'au moins 5 à 10°C à la température de
stabilité de la micro-émulsion lorsque le milieu aqueux
est constitué par de l'eau pure.

2. Microémulsion selon la revendication 1, caractérisée en ce que le milieu aqueux est de l'eau pure,
des solutions aqueuses salines ou amylacées, des milieux
aqueux physiologiquement acceptables.

3. Microémulsion selon l'une des revendications 1 ou 2, caractérisée en ce que le fluorocarbure
est un perfluorocycloalcane, un perfluoroalkylcycloalcane, un perfluoroalcane ou un fluoroalcane partiellement hydrogéné saturé ou insaturé, un produit de formule $C_nF_{2n+1}CH=CH-C_mF_{2m+1}$ , $\underline{n}$ et $\underline{m}$ étant des nombres
entiers; un composé hétérocycliques perfluorés alkyl-
substitués, une amine tertiaire perfluorée, un éther
perfluoré.

4. Microémulsion selon l'une quelconque des
revendications 1 à 3, caractérisée en ce que le fluorocarbure est choisi parmi les composés ci-après :

perfluorodécaline,

perfluorotributylamine,

perfluorotripropylamine,

perfluorométhyldécaline,

perfluoroadamantane,

perfluorodiméthyladamantane et

perfluorodécane, le composé de formule $C_8F_{17}CH=CH_2$
ou le composé de formule $C_8F_{17}CH_2CH_3$.

5. Microémulsion selon l'une quelconque des revendications 1 à 4, caractérisée en ce que l'agent tensio-actif fluoré non ionique est un composé de formule

$R_F CH_2(OC_2H_4)_n OH$

dans laquelle $R_F$ est une chaîne fluorocarbonée ou fluorocarbonée partiellement hydrogénée.

$\underline{n}$ est un nombre entier au moins égal à 1.

6. Microémulsion selon l'une quelconque des revendications 1 à 5, caractérisée en ce que l'agent tensio-actif est l'un des composés ci-après :

$C_7F_{15}CH_2(OC_2H_4)_4 OH$; $C_7F_{15}CH_2 (OC_2H_4)_5 OH$; $C_7F_{15}CH_2 (OC_2H_4)_6 OH$; $C_6F_{13}CH_2(OC_2H_4)_3 OH$; $C_6F_{13}CH_2(OC_2H_4)_4 OH$; $C_6F_{13}CH_2(OC_2H_4)_5 OH$ et $C_6F_{13}CH_2 (OC_2H_4)_6 OH$.

7. Microémulsion selon l'une quelconque des revendications 1 à 6, stable à environ 37°C, caractérisée en ce qu'elle comprend :

environ 0 à 55% de perfluorodécaline environ 0 à 15% de l'agent tensio-actif de formule $C_7F_{15}CH_2(OC_2H_4)_5 OH$, le complément étant de l'eau.

8. Microémulsion selon l'une quelconque des revendications 1 à 6 stables à environ 37°C, caractérisée en ce qu'elle comprend en poids environ 0 à 60% de $C_8F_{17}CH{=}CH_2$

environ 0 à 20% de l'agent tensio-actif de formule $C_7F_{15}CH_2(OC_2H_4)_6 OH$, le complément étant l'eau.

9. Microémulsion selon l'une quelconque des revendications 1 à 6, stable à 37°C, caractérisée en ce qu'elle comprend :

environ 0 à55% de $C_8F_{17}CH{=}CH_2$

environ 0 à 20% de $C_7F_{15}CH_2(OC_2H_4)_6 OH$

le reste étant constitué par la solution de Ringer lactate.

10. Microémulsion selon l'une quelconque des revendications 1 à 6, stable à 37°C, caractérisée en ce qu'elle contient :

environ 0 à 45% de perfluorodécaline,

environ 0 à 50% de $C_6F_{13}CH_2(OC_2H_4)_5OH$ le reste étant constitué par de l'eau.

11. Microémulsion selon l'une quelconque des revendications 1 à 6 stable à 25°C, caractérisée en ce qu'elle comprend :

0 à 45% de perfluorodécaline,

0 à 50% de $C_6F_{13}CH_2(OC_2H_4)_5$ OH

le reste étant constitué par de l'eau.

12. Procédé pour l'obtention d'une microémulsion selon l'une quelconque des revendications 1 à 11, caractérisé en ce qu'il consiste à déterminer au préalable la zone de concentration spécifique de la température de stabilité désirée, à mélanger le fluorocarbure, un milieu aqueux et l'agent tensio-actif dans des quantités appropriées comprises dans ladite zone de concentration et à chauffer le mélange résultant jusqu'à la température de stabilité désirée.

13. Application des microémulsions selon l'une quelconque des revendications 1 à 11, à titre d'agents transporteurs de gaz, tels que $O_2$, $N_2$, $CO_2$ comme agents thérapeutiques ou pour la perfusion d'organes isolés ou comme additifs dans les milieux de culture de bactéries aérobies ou anaérobies.

14. Procédé de traitement thérapeutique, caractérisé en ce qu'il consiste à administrer, lors d'une transfusion sanguine une microémulsion selon l'une quelconque des revendications 1 à 11.

15. Formulation pharmaceutique caractérisée en ce qu'elle comprend une microémulsion selon l'une quelconque des revendications 1 à 11.

FIG.1

PERFLUORODECALINE

37°C

H$_2$O    C$_7$F$_{15}$CH$_2$(OC$_2$H$_4$)$_5$OH

0051526

FIG.2

$C_8F_{17}CH = CH_2$

$C_7F_{15}CH_2(OC_2H_4)_6OH$

$H_2O$

37°C

25°C

0051526

FIG.3

$C_8F_{17}CH=CH_2$

90

80

70

60

50

37°C

40

30

20

10

SOLUTION
LACTÉE DE RINGER

$C_7F_{15}CH_2(OC_2H_4)_6OH$

0051526

FIG. 4

PERFLUORODECALINE

37°C
25°C

$H_2O$

$C_6F_{13}CH_2(OC_2H_4)_5OH$

FIG.5

FIG.6

PERFLUORODECALINE

90

80

70

60

50

37°C

40

30

20

10

H₂O
OU
PLASMION

PLASMION

H₂O

$C_7 F_{15}CH_2(OC_2H_4)_6 OH$

6/6

0051526

**Office européen des brevets**

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

0051526
Numéro de la demande

EP 81 40 1690

## DOCUMENTS CONSIDERES COMME PERTINENTS

| Catégorie | Citation du document avec indication, en cas de besoin, des parties pertinentes | Revendication concernée |
|---|---|---|
| XD | CHEMICAL ABSTRACTS, vol. 83, no. 2, 14 juillet 1975, page 349, abrégé 15661d Columbus, Ohio, U.S.A. | |
| | & JP - A - 74 123 184 (ASAHI DENKA KOGYO K.K.) 25-11-1974 | |
| | * En entier * | 1-13, 15 |
| | -- | |
| XD | US - A - 3 778 381 (HENRI L. ROSANO et al.) | |
| | * Colonne 2, ligne 36 - colonne 4, ligne 76 * | 1-4 |
| | -- | |
| D | FR - A - 2 105 287 (THE GREEN CROSS CORPORATION ET TANABE SEIYAKU CO. LTD.) | |
| | * Page 12; revendications * | 1,3,4 |
| | -- | |
| D | FR - A - 2 313 024 (SON VENTURES et al.) | |
| | * Page 9, revendications * | 1 |
| | -- ./. | |

**CLASSEMENT DE LA DEMANDE (Int. Cl.³)**

A 61 K   9/00
         31/02
         31/025
         31/08
         31/13
C 12 N   1/00

**DOMAINES TECHNIQUES RECHERCHES (Int. Cl.³)**

A 61 K   9/00
         31/02
         31/025
         31/08
         31/13

### RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:   1-13, 15
Revendications ayant fait l'objet de recherches incomplètes:
Revendications n'ayant pas fait l'objet de recherches:   14: Méthode de
Raison pour la limitation de la recherche:   traitement chirurgical ou thérapeutique du corps humain ou animal. Voir article 52(4) de la convention sur le brevet européen.

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

| Lieu de la recherche | Date d'achèvement de la recherche | Examinateur |
|---|---|---|
| La Haye | 28-01-1981 | REMPP |

OEB Form 1505.1  06.78